# EUROPEAN PATENT APPLICATION

(11) **EP 1 698 274 A1**
(43) Date of publication of application: **06.09.2006**
(21) Application number: 04818975.7
(22) Date of filing: 19.11.2004
(51) Int. Cl.: A61B 5/00

(54) **HEALTH DATA COLLECTION APPARATUS**

(30) Priority: 20.11.2003 JP 2003390537
(71) Applicant: MATSUSHITA ELECTRIC INDUSTRIAL CO., LTD., Kadoma-shi, Osaka 571-8501 (JP)
(72) Inventor: TANAKA, Toshiyuki c/o Matsushita Electric, 3-7, Shiromi 1-chome, Chuo-ku, Osaka-shi (JP); NAGAI, Kazutoshi c/o Matsushita Electric, 3-7, Shiromi 1-chome, Chuo-ku, Osaka-shi (JP); AKAI, Naruaki c/o Matsushita Electric, 3-7, Shiromi 1-chome, Chuo-ku, Osaka-shi (JP)
(74) Representative: Eisenführ, Speiser & Partner
(86) International application number: PCT/JP2004/017251
(87) International publication number: WO 2005/048833

(57) **Abstract**

A health data collection system for higher quality health care is provided. A patient terminal 21 has a data processor 34 for storing, in a memory 33, vital data obtained by a vital sensor 25 and voice data inputted through a microphone 27 in a memory 33in association with each other. The vital data and voice data stored in the memory 33 are transmitted to a center server 22 via a communication network 24 and accumulated in a database server40. A doctor terminal 23 has a memory 52 for storing the vital data and voice data received from the database server 40, and a data processor 57 for outputting the vital data and voice data stored in the memory 52 to a CRT display 55 and speaker 53 in association with each other.

## Description

### Technical Field

The present invention relates to a health data collection system in which data on health of patients, such as a blood pressure or blood glucose level, is collected at home, for example, and the data is available by health care providers such as doctors so that the health care providers can remotely perceive the health condition of the patient.

### Background Art

Conventionally, this type of health data collection system includes a data input unit and communication unit, and communicates with an external communication destination via a communication line (See Patent Document 1).

Fig. 14 illustrates the health data collection system disclosed in the Patent Document 1. The health data collection system generally comprises a patient terminal 1 set on the side of a patient as a person to be measured, a center server 3 including a database server 11 as storage means for storing data collected from the patient terminal 1, an application server 10, and a control terminal 12, and a doctor terminal 2 which is for a health care provider such as a doctor to refer to the data stored in the center server 3. The patient terminal 1, center severs 3 and doctor terminal 2 are connected to each other via a communication network 4.

The patient terminal 1 includes vital sensors 5 such as a thermometer and blood pressure meter for collecting vital data of a patient, a storage unit (not shown) for storing the data collected by the vital sensors 5, and anoperationunit (not shown) to be operated by the patient. The patient terminal 1 further includes a communication controller 6 for transmitting the data stored in the storage unit to the center server 3 via the communication network 4.

In addition to the above, the patient terminal 1 includes a TV telephone 7 for communicating with the doctor terminal by utilizing image and voice.

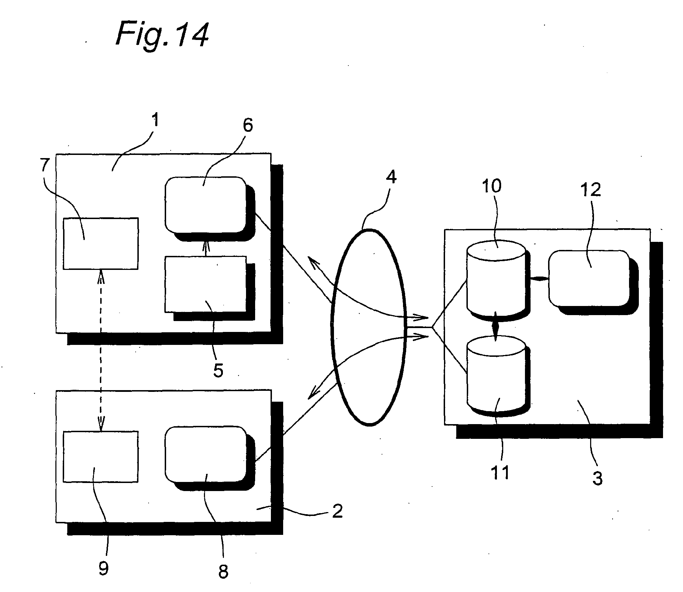

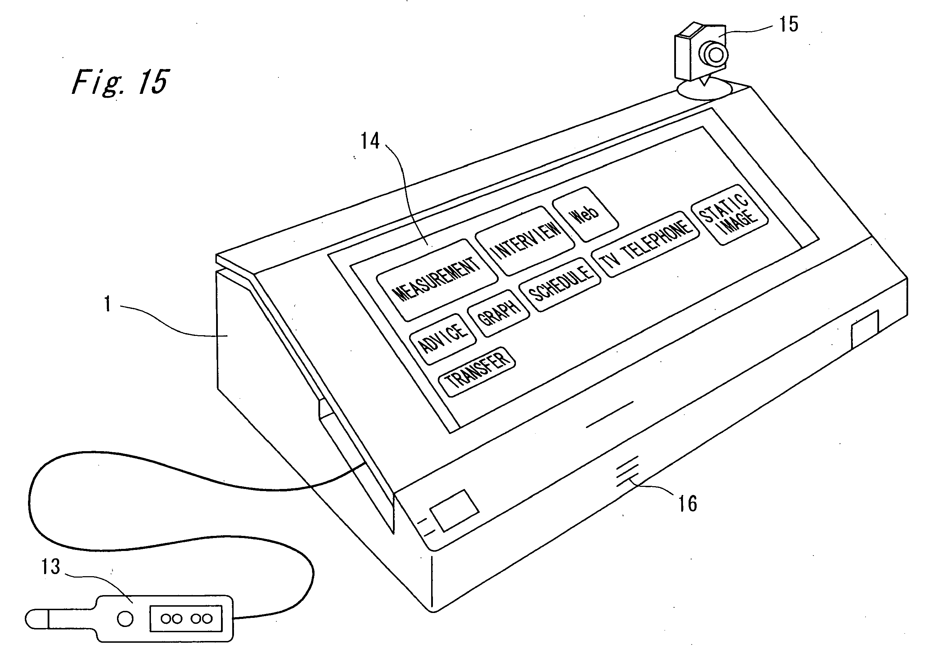

Fig. 15 shows the appearance of an example of apparatus which can be used as the patient terminal 1 of the health data collection system of Fig. 14. Connected to the patient terminal 1 are a liquid crystal display 14 provided with a touch panel to be operated by the patient and a clinical thermometer 13 which is an example of vital sensors 5. Further, the patient terminal 1 includes a microphone 16 for voice input and a camera 15 for image input of the TV telephone 7.

Referring again to Fig. 14, the doctor terminal 2 includes a communication controller 8 which performs the communication with the center server 3 and the display for data reference by the doctor. The doctor terminal 2 further includes a TV telephone 9 for conducting telephone communication with the patient terminal 1.

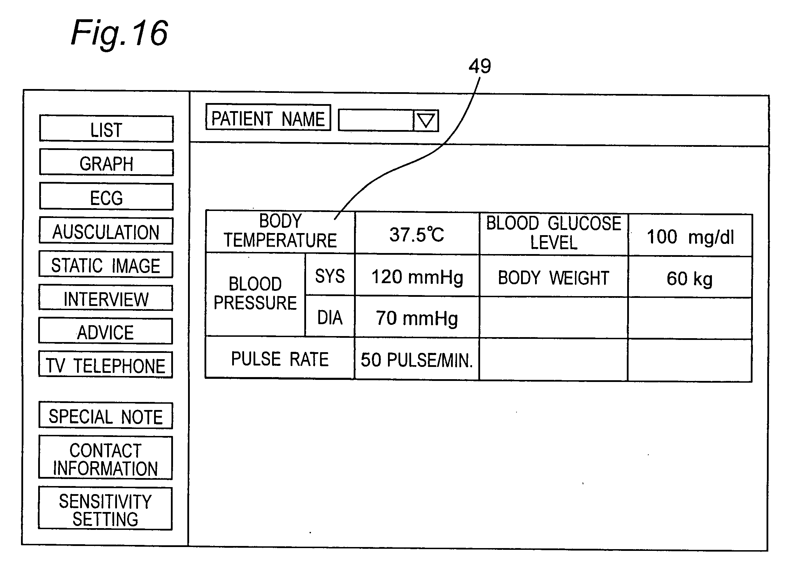

Fig. 16 shows an example of screen display of the communication controller 8 of the doctor terminal 2. When a patient's name is selected on the screen, data such as the body temperature, blood pressure and pulse transmitted from the patient terminal 1 of the selected patient is displayed in the form of a list 49.

Data measured by a patient by using measurement means such as the thermometer 13 is once stored in the communication controller 6 of the patient terminal 1 and then transmitted to the center server 3 via the communication network 4 to be stored in the database server 11 of the center server 3.

When the doctor performs the operation to extract the vital data of a patient from the database server 11 of the center server 3 by the doctor terminal 2, the vital data of that patient is called up from the database server 11 to the communication controller 8 of the doctor terminal 2 via the communication network 4 and appears on the display, as shown in Fig. 16.

From the displayed data, the doctor can perceive the health condition of the patient, and utilize the data as reference materials for medical treatment and diagnosis. Further, if necessary, the doctor can communicate with the patient by image and voice by communicating with the TV telephone 7 of the patient terminal 2 using the TV telephone 9 of the doctor terminal 2.

As discussed above, the conventional health data collection system provides an environment in which the doctor can perceive the health data of a patient even when the doctor and the patient are physically separated so that good health care can be provided even when the doctor and the patient cannot often exchange visits.

However, the conventional health data collection system has the following drawbacks. First, the measurement of the vital data is generally performed at home by each patient himself or herself, and the patient, who is not a medical professional, is likely to have doubts about the measurement method or make mistakes in measuring the vital data.

Secondly, the health care providers such as doctor extract the vital data of the patient by operating the doctor terminal 2 and browse the health condition of the patient while referring to the data on the screen as shown in Fig. 16. In other words, the doctor needs to perceive the condition of the patient based on the numerical values only. However, since the patient may be inexperienced in the measurement as described above, it may not be desirable to diagnose based on the numerical values only. Practically, the doctor often needs to diagnose while taking into consideration the information as to the situation in which the patient has performed the measurement and information as to the matter which the patient is worrying about and the like.

In such a case, the doctor needs to contact the patient to perceive more about the condition of the patient. As the means for establishing contact between the patient and the doctor who are at locations remote from each other, the above-described TV telephones or telephones using a public line may be considered. However, such communication means requires, as a precondition, that both of the patient and doctor are near the respective terminals or telephones in real time. Generally, the timing at which the patient transmits the data differs from the timing at which the doctor checks the data. Therefore, even when the doctor tries to contact the patient, the doctor often cannot contact the patient because of the absence of the patient. In such a case, the doctor needs to make great effort to reach the patient by trying to contact the patient many times. Such doctor's effort pays off if the patient is found to be actually in bad condition. However, as noted above, if the cause for which the doctor felt the necessity to contact the patient is just a simple mistake in the measurement by the patient, such doctor's effort becomes futile.

Thirdly, the patient may also wish to convey e.g. his or her impressions on measurement data or questions to the doctor. In such a case, the patient has the same problem as the doctor. As communication means which is not real time, e-mail may be considered. However, although the operation of a personal computer may be easy for the doctor, the patients, who is elderly in many cases, has difficulty in operating an input device such as a keyboard. Further, to facilitate the operation, a relatively simple input device like the liquid crystal display 14 with a touch panel shown in Fig. 15 is utilized in the patient terminal 1. Therefore, communication by text by using the patient terminal 1 is very difficult.

Patent Document 1: Japanese Patent Application Laid-open Publication No. No.2002-83066

### Disclosure of the Invention

### Problems Solved by the Invention

An object of the present invention is to provide a health data collection system which facilitates remote health care by adding new information to the vital data measured by a patient. Other object of the present invention is to provide a health data collection system which enhances the quality of health care by smoothing the communication between a patient and health care provider such as a doctor.

### Means for Solving the Problems

A first aspect of the present invention provides a health data collection system comprising, a measurement unit for obtaining vital data, an input unit for inputting voice data and/or image data, a first data processor for associating the vital data obtained by the measurement unit and the voice data and/or image data inputted by the input unit with each other, a first storage unit for storing the vital data and the voice data and/or image data associated with each other by the first data processor, a first communication unit for transmitting, via a communication network, the vital data and the voice data and/or image data associated with each other and stored in the first storage unit, an accumulation unit for accumulating the vital data and the voice data and/or image data associated with each other and transmitted by the first communication unit, a second communication unit for receiving the vital data and the voice data and/or image data associated with each other from the storage unit via the communication network, and a first output unit for outputting the vital data and the voice data and/or image data received by the second communication unit in association with each other.

The information from a patient, which is the voice data and/or image data inputted by the person to be measured by using the input unit, is associated with the vital data obtained by the measurement unit and stored in the first storage unit by the first data processor. The vital data and the voice data and/or image data stored in the first storage unit are transmitted by the first communication unit, via the communication network, to the accumulation unit for accumulating therein. The vital data and the voice data and/or image data stored in the accumulation unit are transmitted to the second communication unit via the communication network. Further, the vital data and the voice data and/or image data are outputted in association with each other by the first output unit. By this arrangement, new information in the form of voice or image can be added by the patient to the vital data and transmitted to a health care provider at a remote location. Therefore, the health care provider can effectively utilize each piece of vital data. Thus, when the health care provider makes a diagnosis, doubt due to the lack of information from the patient's side can be reduced. Further, the health care provider can save the trouble of confirmation work which the health care provider needs to perform to clear such doubt. As a result, the remote health care is facilitated and the quality thereof is enhanced. Moreover, since information from the patient's side can be transmitted in the form of voice and image in association with vital data, the communication between the patient and the health care provider becomes smooth, whereby good remote health care can be realized.

The first data processor adds information indicating that the vital data is associated with the voice data and/or image data to the vital data, and stores the vital data in the first storage unit. By referring to the vital data, the voice data and/or image data associated with the vital data can be checked. Therefore, new or another data for the association is not necessary, so that the association can be performed easily.

Alternatively, the first data processor adds information indicating that the voice data and/or image data is associated with the vital data to the voice data and/or image data, and stores the voice data and/or image data in the first storage unit. In referring to the voice data or image data, the associated vital data can be easily referred to. Therefore, the health care provider can easily check only the matter which the patient wishes to report to the health care provider or the vital data which the patient cares about, whereby efficient data check by the health care provider can be realized.

The health data collection system may further comprise a second output unit for outputting the vital data and the voice data and/or image data. In this arrangement, the first data processor extracts the vital data and the voice data and/or image data from the first storage unit, associates the vital data and the voice data and/or image data with each other, and has the second output unit output the vital data and the voice data and/or image data. The patient can confirm the voice data and/or image data added to the measured vital data by the patient himself or herself. In other words, the patient can confirm the information transmitted by himself or herself, and further can refer to the vital data measured by himself or herself together with the associated voice data and/or image data as history or record and utilize for his or her own health care.

Preferably, the health data collection system may further comprise a second storage unit for storing the vital data and the voice data and/or image data associated with each other and received by the second communication unit, and a second data processor for extracting the vital data and the voice data and/or image data stored in the second storage unit, associating the vital data and the voice data and/or image data with each other, and having the first output unit output the vital data and the voice data and/or image data.

A second aspect of the present invention provides a patient terminal of a health data collection system. Specifically, the second aspect of the present invention provides a terminal of a health data collection system comprising, an operation unit operated by a person to be measured, a measurement unit for obtaining vital data, an input unit for inputting voice data and/or image data, a data processor for associating the vital data obtained by the measurement unit and the voice data and/or image data inputted by the input unit with each other, a storage unit for storing the vital data and the voice data and/or image data associated with each other by the data processor, and a communication unit for transmitting, via a communication network, the vital data and the voice data and/or image data associated with each other and stored in the storage unit.

The patient terminal may further comprise an output unit for outputting the vital data and the voice data and/or image data. In this arrangement, the data processor extracts the vital data and the voice data and/or image data from the storage unit, associates the vital data and the voice data and/or image data with each other, and has the output unit output the vital data and the voice data and/or image data.

The operation unit of the patient terminal may comprise a touch panel. Input means such as a keyboard which is difficult for elderly people to operate is not required, and information such as voice or image can be transmitted by the easy operation of touching the screen itself. Thus, the operation unit can be easily used even by elderly people.

Alternatively, the operation unit of the patient terminal may comprise push buttons. Input means such as a keyboard which is difficult for elderly people to operate is not required, and information such as voice or image can be transmitted by the easy operation of pushing the push buttons. Thus, the operation unit can be easily used even by elderly people.

A third aspect of the present invention provides a health care provider terminal of a health data collection system. Specifically, the third aspect of the present invention provides a terminal of a health data collection system comprising, a communication unit for receiving, via a communication network, vital data and voice data and/or image data stored in association with each other, a storage unit for storing the vital data and the voice data and/or image data associated with each other and received by the communication unit, an output unit for outputting the vital data and the voice data and/or image data, and a data processor for extracting the vital data and the voice data and/or image data stored in the storage unit, associating the vital data and the voice data and/or image data with each other, and having the output unit output the vital data and the voice data and/or image data.

### Effect of the Invention

The health data collection system of the present invention can store voice data and/or image data associated with vital data, and output the vital data and the voice data and/or image data in association with each other. Therefore, practical information can be transmitted from the patient's side, so that the amount of data obtained from each piece of vital data is considerably increased. This facilitates remote health care and realizes smooth communication between patients and health care providers such as doctor.

### Brief Description of Drawings

Fig. 1 is a block diagram of a health data collection system according to a first embodiment of the present invention;
Fig. 2 is a block diagram of a patient terminal in the first embodiment of the present invention;
Fig. 3 is a block diagram of a doctor terminal in the first embodiment of the present invention;
Fig. 4 is a screen image view of the patient terminal of the health data collection system in the first embodiment of the present invention;
Fig. 5 is a data structure view of the health data collection system in the first embodiment of the present invention;
Fig. 6 is a screen image view of the doctor terminal of the health data collection system in the first embodiment of the present invention;
Fig. 7 is a screen image view of the patient terminal of the health data collection system in the first embodiment of the present invention;
Fig. 8 is a block diagram of a patient terminal of a health data collection system according to a second embodiment of the present invention;
Fig. 9 is a screen image view of the patient terminal of the health data collection system in the second embodiment of the present invention;
Fig. 10 is a screen image view of a doctor terminal of the health data collection system in the second embodiment of the present invention;
Fig. 11 is a screen image view of the patient terminal of the health data collection system in the second embodiment of the present invention;
Fig. 12 shows the appearance of a health data collection system in a third embodiment of the present invention;
Fig. 13 is a screen image view of a patient terminal of the health data collection system in the third embodiment of the present invention;
Fig. 14 is a block diagram of a conventional health data collection system;
Fig. 15 shows the appearance of the conventional health data collection system; and
Fig. 16 is a screen image view of a doctor terminal of the conventional health data collection system.

### Explanation of Symbols

- 21: patient terminal
- 22: center server
- 23: doctor terminal
- 24: communication network
- 25: vital sensor
- 27: microphone
- 28: voice input circuit
- 31: liquid crystal display
- 33, 52: memory
- 34, 57: data processor
- 35: touch panel
- 36, 41, 51: communication controller
- 38: camera
- 39: image input circuit
- 40: database server
- 55: CRT display
- 32, 56: image output circuit
- 58: operation unit
- 64: voice record button
- 74, 79: voice memo button
- 35: voice memo button
- 164: image capture button
- 172, 174, 179: image memo button

### Best Mode for Carrying Out the Invention

### (First Embodiment)

A health data collection system according to the present invention will be described below based on the embodiments.

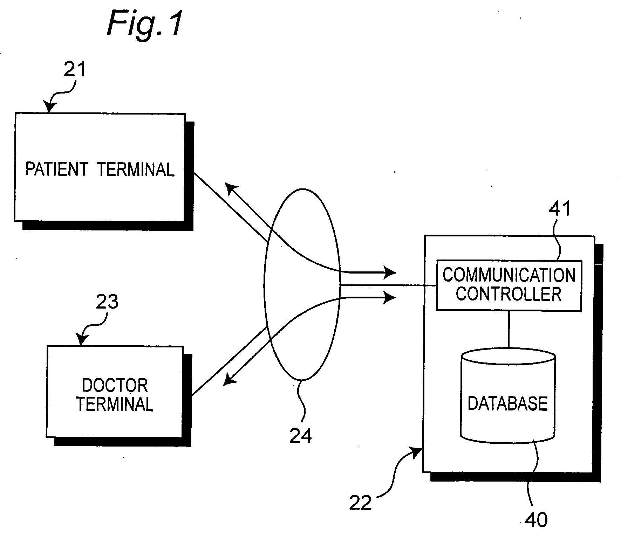

Fig. 1 shows a block diagram of the health data collection system according to a first embodiment of the present invention. The health data collection system generally comprises a patient terminal 21 set on the side of a patient as a person to be measured, a center server 22 as an accumulation unit for accumulating data collected from the patient terminal 21, and a health care provider terminal or a doctor terminal 23 which is for a health care provider such as a doctor to refer to the data stored in the center server 22. The patient terminal 21, the center sever 22, and the doctor terminal 23 are connected to each other via a communication network 24.

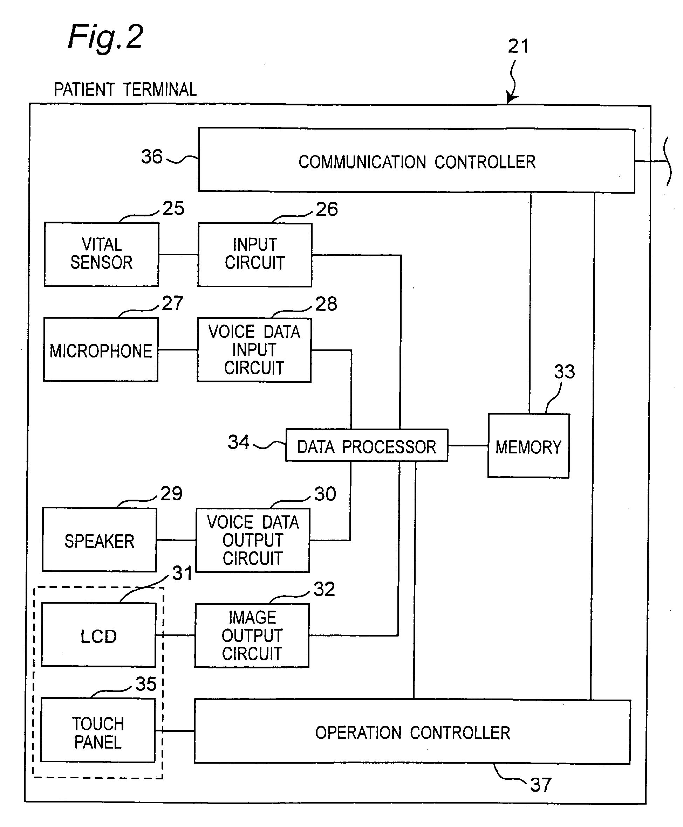

Further referring to Fig. 2, the patient terminal 21 includes vital sensors (measurement unit) 25 such as a clinical thermometer, blood pressure meter and the like as measurement means for collecting vital data of a patient, an input circuits 26 for the vital sensors 25, a microphone (input unit) 27 for voice input by the patient, and a voice input circuit 28. The patient terminal 21 further includes a speaker (output unit) 29, a voice output circuit 30 for the speaker 29, a liquid crystal display (output unit) 31, and an image output circuit 32 for the liquid crystal display 31.

Further, the patient terminal 21 includes a memory (storage unit) 33 for storing the vital data measured by the vital sensors 25 and the voice data which is the voice of the patient inputted through the microphone 27. The patient terminal 21 further includes a data processor 34. The data processor has two functions. As the first function, the data processor 34 associates the vital data from the vital sensors 25 and the voice data from the microphone 27 with each other and stores into the memory 33. As the second function, the data processor 34 extracts the vital data and the voice data in association with each other from the memory 33, and has the liquid crystal display 31 and the speaker 29 output them. Furthermore, the patient terminal 21 includes a touch panel (operation unit) 35 integrally provided on the liquid crystal display 31. A communication controller 36 executes communication with the center server 22 via the communication network 24, which includes transmission of the vital data and voice data to the center server 22. An operation controller 37 controls operations of the entire patient terminal 21 including the communication controller 36 and the data processor 34 based on the operation or instruction inputted by the patient through the touch panel 35, for example.

Referring to Fig. 1, the center server 22 includes a database server 40 and a communication controller 41. The database server 40 accumulates various data including the vital data and the voice data received from the communication controller 36 of the patient terminal 21. The communication controller 41 executes various communications with the patient terminal 21 and the doctor terminal 22, which include reception of the vital data and the voice data from the patient terminal 21 and transmission of the vital data and the voice data to the doctor terminal 22.

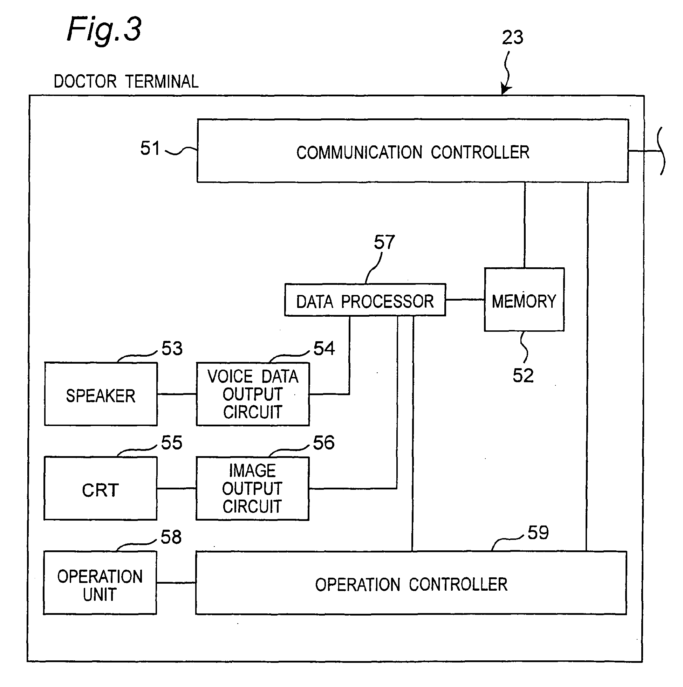

Further referring to Fig. 3, the doctor terminal 23 includes a communication controller 51 for executing communication with the center server 22 via the communication network 24, which includes reception of the vital data and the voice data from the center server 22. Further, the doctor terminal 23 includes a memory (storage unit) 52 for storing the vital data and the voice data received by the communication controller 51. Furthermore, the doctor terminal 23 includes a speaker 53, a voice output circuit 54 for the speaker 53, a CRT display (output unit) 55, and an image output circuit 56 for the CRT display 55. The data processor 57 extracts the vital data and the voice data stored in the memory 52, associates the two kinds of data with each other, and has the speaker 53 and the CRT display 55 output them.

The doctor terminal 23 further includes an operation unit 58, which may comprise a keyboard, mouse and the like, for the operation of the doctor terminal 2 by a health care provider such as a doctor. An operation controller 59 controls operations of the entire doctor terminal 23 including the communication controller 51 and the data processor 57.

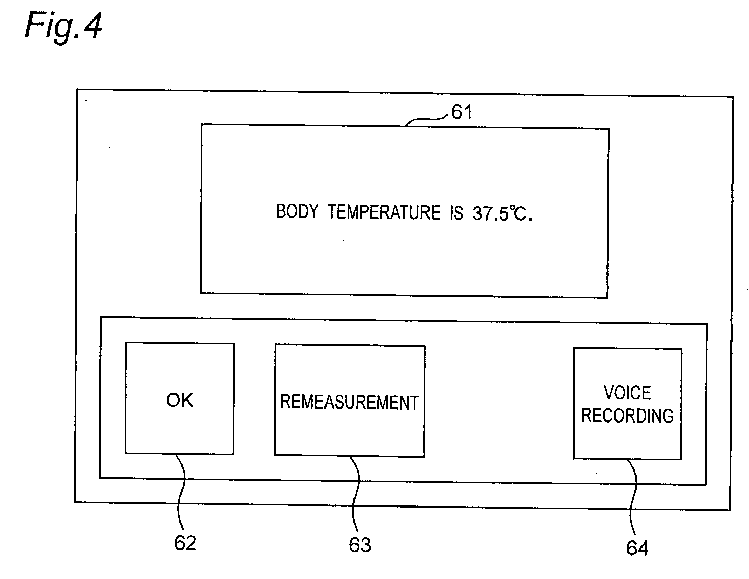

The operation of the health data collection system according to the present embodiment will be described below. Fig. 4 shows an example of the screen of the liquid crystal display 31 of the patient terminal 21 of the health data collection system according to the embodiment. This screen is displayed immediately after the body temperature is measured by using the thermometer which is an example of vital sensors 25. The screen includes a temperature display area 61 at which the measured body temperature (37.5°C) inputted from the thermometer through the input circuit 26 is displayed. When the patient pushes an "OK" button 62, the temperature data is fixed and stored in the memory 33 through the data processor 34. When the patient wishes to measure again, the patient can push a remeasurement button 63 to clear the temperature data currently displayed at the temperature display area 61 and perform remeasurement.

The patient can add some information as the voice data to the measured vital data (body temperature data in this example). In this example, the body temperature (37.5°C) displayed at the temperature display area 61 is slightly higher than normal body temperature. The patient took a bath immediately before measuring the data, because of which the temperature rose slightly. Although the patient recognized that body temperature would rise due to such a reason, the patient performed the measurement, because the patient was required to measure body temperature every day at that time and thought that the lack of data was not desirable. In such a case, it is preferable to input additional information by voice.

Specifically, the patient pushes a voice record button 64 and inputs voice through the microphone 27. In this example, the patient speaks into the microphone 27, "I took a bath immediately before measuring this.", for example. The voice signal inputted through the microphone 27 is transmitted through the voice input circuit 28 and the data processor 34 to be stored in the memory 33, similarly to the vital data (body temperature data in this example).

The voice data is not stored in the memory 33 as it is but stored in the memory 33 as associated with the vital data by the data processor 34. In this embodiment, the data processor 34 adds, to the vital data, information indicating that the vital data is associated with voice data and stores into the memory 33. Conversely, the data processor 34 may add, to the voice data, information indicating that the voice data is associated with vital data, and store into the memory 33.

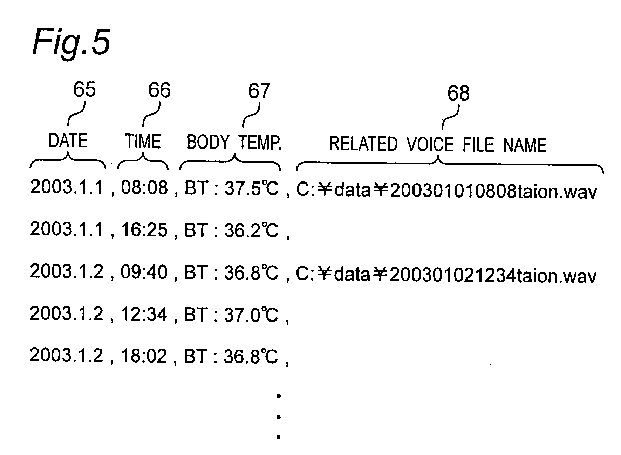

Fig. 5 shows the data structure of the vital data (body temperature data) in this embodiment. Each piece of the body temperature data includes an associated voice file name area 68 for storing the name of the voice file recorded by the patient, in addition to a date area 65, time area 66, and temperature area 67. For example, the piece of temperature data measured at 08:08 on January 1, 2003 is 37.5°C, and the name of the associated voice file is "20031010808taion.wav".

As described above, the vital data and the voice files associated therewith are stored in the memory 33 of the patient terminal 21. When the patient inputs a data transmission command into the patient terminal 21 by operating the touch panel 35, the communication controller 36 reads out the appropriate vital data (body temperature data in this example) and the voice data associated therewith from the memory 33 and transmits them to the center server 22 via the communication network 4. The vital data and the voice data associated therewith are received by the communication controller 41 of the center server 22 and stored in the data base server 40.

The doctor can extract data of the appropriate patient by operating the doctor terminal 2. Specifically, when the doctor inputs a reception command into the doctor terminal 23 by operating the operation unit 58 of the doctor terminal 2, reception request is transmitted from the communication controller 51 of the doctor terminal 2 to the center server 22 via the communication network 24. When the communication controller 41 of the center server 22 receives the reception request, the appropriate vital data is extracted from the database server 40. The communication controller 40 transmits the extracted vital data to the doctor terminal 23 via the communication network 24.

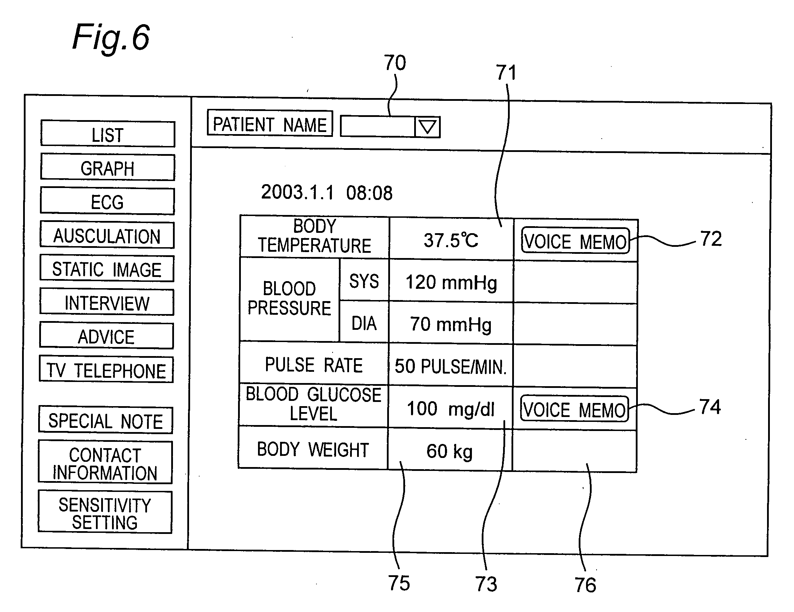

The vital data is received by the communication controller 51 of the doctor terminal 23 and stored in the memory 52. Further, the received vital data 51 is displayed at the CRT display 55 via the data processor 57 and the image output circuit 56. Fig. 6 shows an example of screen displayed on the CRT display 55 of the doctor terminal 23. When the doctor designates the name of a patient at a drag menu 70, the vital data of the patient is displayed in the form of a list. The body temperature data described before is displayed at a temperature data area 71.

When vital data to be displayed at the CRT display 55 is associated with voice data, the data processor 57 displays a voice memo button at an area on the right side of the vital data. As described before, in the example of Fig. 6, since voice information on the body temperature data is recorded by the patient, a voice memo button 72 is displayed at the area on the right side of the temperature data area 71. Similarly, a voice memo button 74 is also displayed at the area on the right side of a blood glucose level area 73 including blood glucose level. On the other hand, the area on the right side of a weight area 75 including body weight data is kept blank 76, because the body weight data is not associated with voice data.

When the doctor clicks the voice memo button 33 on the right side of the temperature area 71 in Fig. 6, the data processor 57 refers to the temperature data (Fig. 5) in the memory 52 and obtains the name of the voice file associated with that temperature data. Subsequently, the data processor 57 accesses the database server 40 via the communication controller 51 of the doctor terminal 23, the communication network 24, and the communication controller 41 of the center server 22 to retrieve the database server 40. As a result, the voice file associated with that temperature data is downloaded to the memory 52 of the doctor terminal 23. The voice data stored in the downloaded voice file is outputted from the speaker 53 via the data processor 57 and the voice output circuit 54.

In this example, the voice message recorded by the patient, i.e., "I took a bath immediately before measuring this." is replayed through the speaker 53. By this information, the doctor can immediately perceive that the relatively high temperature of 37.5°C is caused by the bath immediately before the body temperature measurement, not by some problem in health condition. Therefore, the doctor need not particularly confirm the temperature data, and no special labor is required. The doctor may be kind enough to give proper advice about the measurement. In this case, since the situation is not urgent, communication means such as e-mail can be used. In such a case, smooth communication can be established without forcing both of the person to be measured and the doctor to spend time.

At the blood glucose level area 73 of Fig. 6, the blood glucose level data of 100mg/dl is displayed, and the voice memo button 74 corresponding to the blood glucose area 73 is displayed, as described above. When the voice memo button 74 is clicked, the patient' s voice is replayed through the speaker 53. For example, in the case where the patient recorded the question that says, "Is the data normal?", the question is replayed through the speaker 53. Therefore, the doctor can immediately recognize the patient's question and make a proper reply to the question.

In this way, according to the health data collection system of the present embodiment, information can be transmitted from the patient' s side to the doctor' s side as voice data associated with vital data, so that the doctor can send back proper advice about the information.

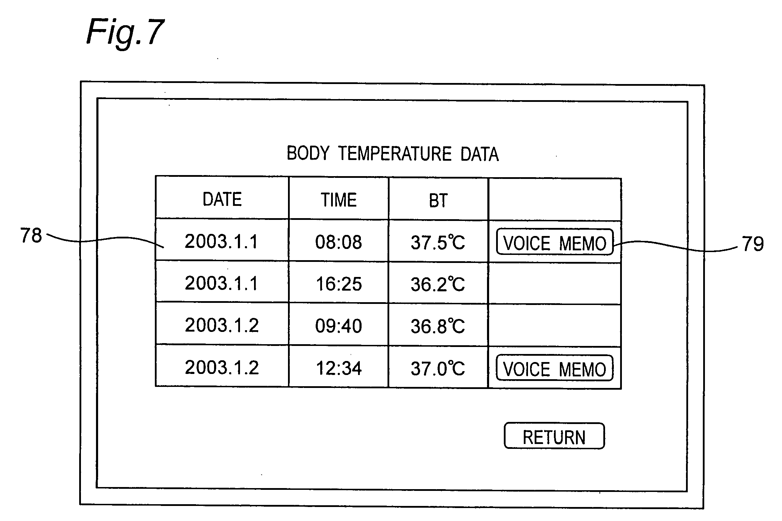

The patient can replay the voice recorded by himself or herself by using the patient terminal 21. Fig. 7 shows an example of temperature data confirmation screen displayed at the liquid crystal display 31 of the patient terminal 21. In the figure, the body temperature data stored in the memory 33 is displayed together with date and time in the form of a list. When any piece of temperature data is associated with voice data, the data processor 34 displays a voice memo button 79 at an area on the right side of the data display area 78 of that piece of temperature data. For example, since the piece of temperature data measured at 08:08 on January 1, 2003 is associated with voice data, a voice memo button 79 is displayed on the right side of the temperature display area 78, similarly to the screen (See Fig. 6) of the doctor terminal 23.

When the patient pushes the voice memo button 79 in Fig. 7, the data processor 34 searches the memory 33. As a result, the voice data associated with that piece of temperature data is outputted from the speaker 29 via the voice output circuit 30. Therefore, the patient can check the voice data recorded by himself or herself just by pushing the voice memo button 79.

In this way, in the health data collection system of this embodiment, the patient terminal 21 records the vital data and the relevant voice data in association with each other and outputs them in association with each other to the center server 22 so that the doctor can browse the vital data along with the associated voice data by the doctor terminal 23. Therefore, practical information can be transmitted from the patient's side to the doctor' s side, so that the amount of data the doctor can obtain from each piece of vital data is considerably increased. Therefore, for the doctor, a judgment which is difficult to make based on only the vital data can be made considerably easily by the combination of the vital data with voice data. As a result, waste of labor by the doctor can be reduced, resulting in that the doctor can focus on necessary works.

Further, for a patient, a trivial question which the patient hesitates to ask on the phone or the like can be easily sent to the doctor by transmitting the information. Therefore, the doctor and the patient can smoothly communicate with each other, whereby the trustful relationship between the patient and doctor can be enhanced. In this way, the quality of health care at remote locations can be considerably enhanced.

Further, since a relatively simple interface such as the touch panel 35 is used in the patient terminal 21, it is unnecessary to force elderly people to operate a relatively difficult input device such as a keyboard, mouse, and the like.

### (Second Embodiment)

A health data collection system a second embodiment of the present invention differs from that of the first embodiment in that, in addition to voice data, image data can be added to vital data.

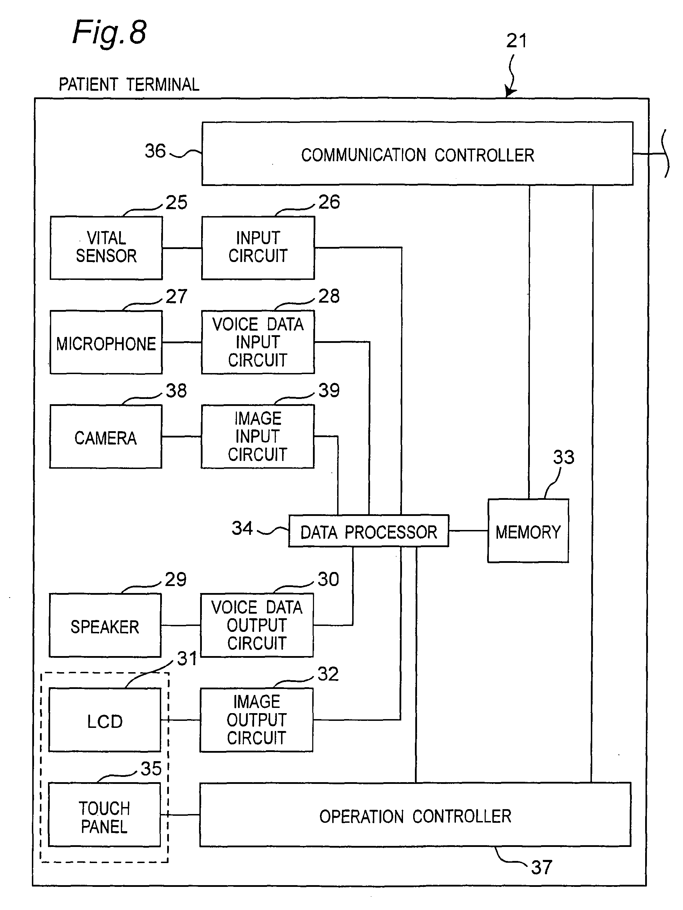

Referring to Fig. 8, the patient terminal 21 of the second embodiment includes a camera 38 for image capturing and an image input circuit 39 for the camera 38. The data processor 34 has a function to associate the vital data from the vital sensor 25 with the image data from the camera 38 and the voice data from the microphone 27 and stores in the memory 33. Further, the data processor 34 has a function to extract the vital data and the associated image data and the voice data stored in the memory 33 and has the liquid crystal display 31 and the speaker 29 output them.

The data processor 57 (See Fig. 3) of the doctor terminal 23 of the second embodiment extracts the vital data, the voice data, and the image data stored in the memory 52, associates these data with each other and has the speaker 53 and the CRT display 55 output these data.

Further, the database server 40 of the center server 22 according to the second embodiment stores various kinds of data including the vital data, the voice data, and the image data received from the communication controller 36 of the patient terminal 21.

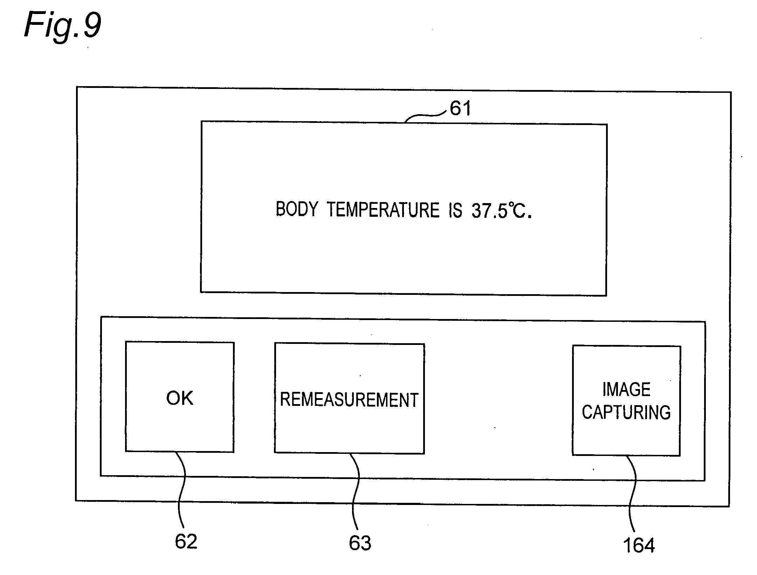

Fig. 9 shows an example of a screen of the liquid crystal display 31 of the patient terminal 21 of the health data collection system according to the present embodiment. Temperature data is displayed on the temperature display area 61. When the "OK" button 62 is pushed, the temperature data is fixed. When the remeasurement button 63 is pushed, the remeasurement becomes possible. The patient pushes an image capture button 164 and inputs voice through the microphone 27 while facing the camera 38 to perform the operation. The image captured by the camera 38 and the voice inputted through the microphone 27 are stored in the memory 33 while being associated with the vital data (body temperature data in this example) by the data processor 34. In this way, the patient can add information as image and voice to the vital data.

The vital data and the voice data and image data associated with the vital data are transmitted from the patient terminal 21 to the center server 22 via the communication network 24 and stored in the database server 40.

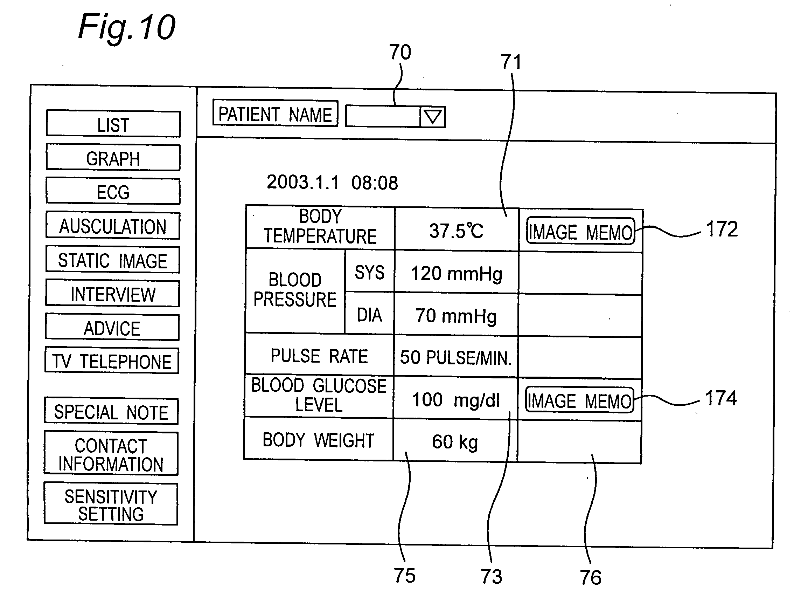

The vital data stored in the database server 40 is received by the doctor terminal 23 and stored in the memory 52. Fig. 10 shows an example of a screen of the doctor terminal 23, which includes a drug menu 70, the temperature area 71, a blood glucose level area 73 and weight area 75. When an image memo button 172 displayed next to the temperature area 71 is clicked, the data processor 57 refers to the temperature data (Fig. 5) in the memory 52 and obtains the name of the voice file and image file associated with that temperature data. Subsequently, the data processor 57 accesses the database server 40 to perform the search. As a result, the voice file and the image data associated with the temperature data is downloaded to the memory 52 of the doctor terminal 23. The voice data stored in the downloaded voice file is outputted from the speaker 53 via the data processor 57 and the voice output circuit 54. The image data stored in the downloaded image file is replayed on the CRT display 55 via the image output circuit 56. When an image memo button 174 displayed next to the blood glucose level area 73 is clicked, the voice data and the image data associated with the blood glucose level data are replayed through the speaker 53 and on the CRT display 55 in a manner similar to the above. In this way, the doctor can browse the patient' s image and voice associated with the vital data by the doctor terminal 23.

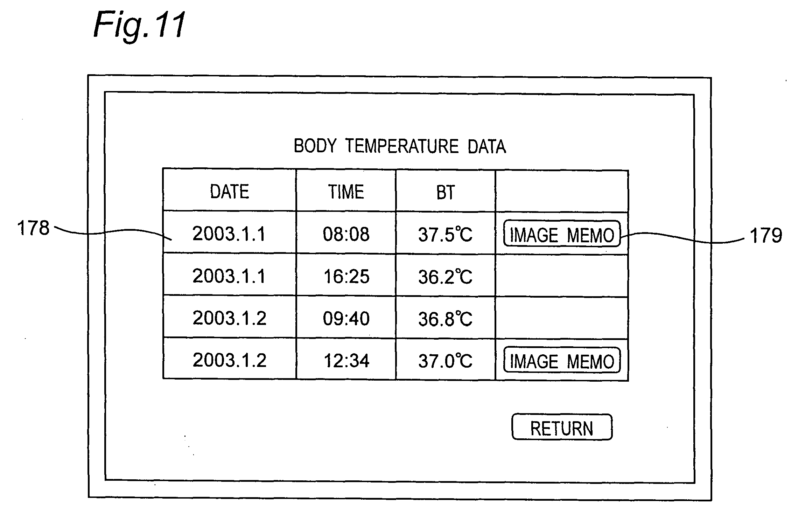

Fig. 11 shows an example of temperature data confirmation screen displayed at the patient terminal 21. When an image memo button 179 on the right side of the data display area 178 for body temperature data is pushed, the data processor 34 searches the memory 33. As a result, the voice data associated with that temperature data is outputted from the speaker 29 via the voice output circuit 30, while the image data associated with that temperature data is replayed on the liquid crystal display 31 via the image output circuit 32. Therefore, the patient can check the voice and image recorded by himself or herself just by pushing the image memo button 179.

### (Third Embodiment)

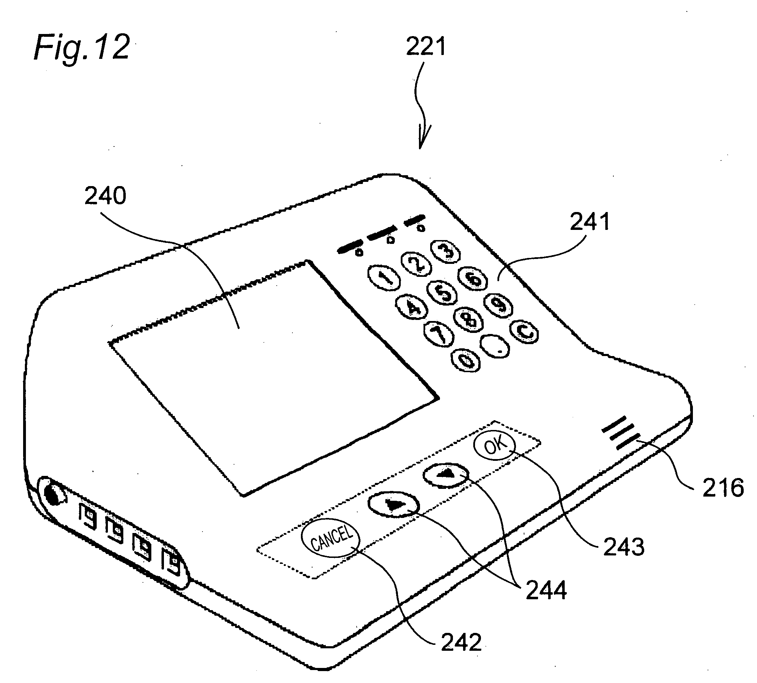

Fig. 12 shows an appearance of a patient terminal 221 of the health data collection system according to a third embodiment of the present invention. The present embodiment differs from the first embodiment in that the terminal does not include a touch panel (a reference sign 35 in Fig. 2), though the terminal includes a liquid crystal display 240. In other words, the liquid crystal display 240 has only a display function.

The patient terminal 221 of the present embodiment includes, as an operation unit replacing the touch panel, up/down buttons 244, an "OK" button 243, a cancel button 242, and numeric buttons 241, all of which are push buttons . Similarly to the patient terminal 21 of the first embodiment, the patient terminal 221 includes a microphone 216 for voice input.

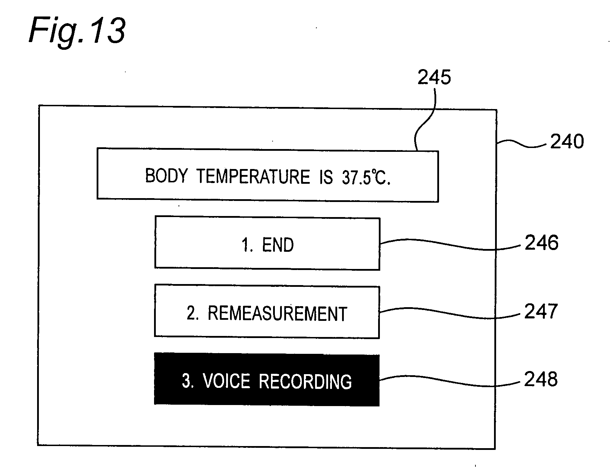

Fig. 13 is an example of a screen of the liquid crystal display 240. Similarly to Fig. 4 of the first embodiment, this example is a screen displayed immediately after temperature is measured. The screen includes a temperature display area 245, an end menu 246, a remeasurement menu 247, and a voice record menu 248.

The basic operation of the patient terminal 1 is performed by selecting a menu item by using the up/down buttons 244 while watching the screen and then confirming the execution of the selectedmenu by using the "OK" button 243. In the selected item, the letter and/or sign appears white on a black background.

In the example of Fig. 13, the voice record menu 248 is selected. When the patient pushes the "OK" button 243 in this state, the recording is started. Therefore, when the patient speaks into the microphone 216, the voice is recorded.

By the combination of the liquid crystal display 240 for performing display of the letter and/or sign and the push buttons 241 to 244 for performing operation, a larger amount of information useful for health care can be inputted from the patient terminal 221, so that good health care service can be achieved. Moreover, since the push buttons 241 to 244 do not require such high level operation as are necessary for operating an input device such as a keyboard, even elderly people can use easily.

Although the present invention has been fully described in conjunction with preferred embodiments thereof with reference to the accompanying drawings, various changes and modifications are possible for those skilled in the art. Therefore, such changes and modifications should be construed as included in the present invention unless they depart from the intention and scope of the invention as defined by the appended claims.

### Industrial Applicability

As described above, according to the health data collection system of the present invention, the quality of health care at remote locations can be considerably enhanced, and the usability in the field of telemedicine is extremely high.

## Claims

1. A health data collection system comprising:
a measurement unit for obtaining vital data;
an input unit for inputting voice data and/or image data;
a first data processor for associating the vital data obtained by the measurement unit and the voice data and/or image data inputted by the input unit with each other;
a first storage unit for storing the vital data and the voice data and/or image data associated with each other by the first data processor;
a first communication unit for transmitting, via a communication network, the vital data and the voice data and/or image data associated with each other and stored in the first storage unit;
an accumulation unit for accumulating the vital data and the voice data and/or image data associated with each other and transmitted by the first communication unit;
a second communication unit for receiving the vital data and the voice data and/or image data associated with each other from the storage unit via the communication network; and
a first output unit for outputting the vital data and the voice data and/or image data received by the second communication unit in association with each other.

2. The data collection system according to claim 1, wherein the first data processor adds information indicating that the vital data is associated with the voice data and/or image data to the vital data, and stores the vital data in the first storage unit.

3. The data collection system according to claim 1, wherein the first data processor adds information indicating that the voice data and/or image data is associated with the vital data to the voice data and/or image data, and stores the voice data and/or image data in the first storage unit.

4. The health data collection system according to any one of claims 1 to 3, further comprising a second output unit for outputting the vital data and the voice data and/or image data,
wherein the first data processor extracts the vital data and the voice data and/or image data from the first storage unit, associates the vital data and the voice data and/or image data with each other, and has the second output unit output the vital data and the voice data and/or image data.

5. The health data collection system according to any one of claims 1 to 4, further comprising:
a second storage unit for storing the vital data and the voice data and/or image data associated with each other and received by the second communication unit; and
a second data processor for extracting the vital data and the voice data and/or image data stored in the second storage unit, associating the vital data and the voice data and/or image data with each other, and having the first output unit output the vital data and the voice data and/or image data.

6. A terminal of a health data collection system comprising:
an operation unit operated by a person to be measured;
a measurement unit for obtaining vital data;
an input unit for inputting voice data and/or image data;
a data processor for associating the vital data obtained by the measurement unit and the voice data and/or image data inputted by the input unit with each other;
a storage unit for storing the vital data and the voice data and/or image data associated with each other by the data processor; and
a communication unit for transmitting, via a communication network, the vital data and the voice data and/or image data associated with each other and stored in the storage unit.

7. The terminal according to claim 6, wherein the data processor adds information indicating that the vital data is associated with the voice data and/or image data to the vital data, and stores the vital data in the storage unit.

8. The terminal according to claim 6, wherein the data processor adds information indicating that the voice data and/or image data is associated with the vital data to the voice data and/or image data, and stores the voice data and/or image data in the storage unit.

9. The terminal according to any one of claims 6 to 8, further comprising an output unit for outputting the vital data and the voice data and/or image data,
wherein the data processor extracts the vital data and the voice data and/or image data from the storage unit, associates the vital data and the voice data and/or image data with each other, and has the output unit output the vital data and the voice data and/or image data.

10. The terminal according to any one of claims 6 to 9, wherein the operation unit comprises a touch panel.

11. The terminal according to any one of claims 6 to 9, wherein the operation unit comprises push buttons.

12. A terminal of a health data collection system comprising:
a communication unit for receiving, via a communication network, vital data and voice data and/or image data stored in association with each other;
a storage unit for storing the vital data and the voice data and/or image data associated with each other and received by the communication unit;
an output unit for outputting the vital data and the voice data and/or image data; and
a data processor for extracting the vital data and the voice data and/or image data stored in the storage unit, associating the vital data and the voice data and/or image data with each other, and having the output unit output the vital data and the voice data and/or image data.
